# EUROPÄISCHE PATENTANMELDUNG

(11) **EP 1 685 826 A1**
(43) Veröffentlichungstag der Anmeldung: **02.08.2006**
(21) Anmeldenummer: 06001712.6
(22) Anmeldetag: 27.01.2006
(51) Int. Cl.: A61K 8/49, A61K 8/63, A61Q 5/00, A61Q 5/02, A61Q 5/12

(54) **Verwendung eines coffeinhaltigen Mittels**

(30) Priorität: 27.01.2005 DE 102005003949
(71) Anmelder: Dr. Kurt Wolff GmbH & Co. KG, 33611 Bielefeld (DE)
(72) Erfinder: Salmina, Manuela, 33098 Paderborn (DE); Klenk, Adolf, Dr., 33415 Verl (DE)
(74) Vertreter: Vièl, Christof

(57) **Zusammenfassung**

Die Erfindung betrifft die Verwendung eines coffeinhaltigen Mittels, das für den kurzzeitigen Kontakt mit der Kopfhaut bestimmt ist.

Um ein Mittel zu schaffen, das in der Anwendung dem Mann bereits gut geläufig ist, so dass die Akzeptanz und damit auch die regelmäßige Anwendung sichergestellt ist., wird im Rahmen der Erfindung die Verwendung eines coffeinhaltigen Mittels, das für den kurzzeitigen Kontakt mit der Kopfhaut bestimmt ist und als Penetrationshilfe anionische und amphotere oberflächenaktive Stoffe (Tenside) sowie die Haarwurzeln stabilisierende Stoffe, insbesondere Minoxidil oder Finasterid, enthält, wobei das Mittel Coffein in einer Menge zwischen 0,01 und 10% enthält und die Konzentration an anionischen oberflächenaktiven . Stoffen (Tenside) zwischen 0,1 und 50% beträgt, vorzugsweise zwischen 1 % und 15%, gegen erblich bedingten Haarausfall vorgeschlagen.

## Beschreibung

Die Erfindung betrifft die Verwendung eines coffeinhaltigen Mittels, das für den kurzzeitigen Kontakt mit der Kopfhaut bestimmt ist.

Die Behandlung von androgenetisch bedingtem Haarausfall ist nach wie vor ein ungelöstes Problem. Durch eine genetische Veranlagung ist festgelegt, dass ab einem bestimmten Zeitpunkt die Haarfollikel im fronto-parietalen und im zentralen Sektor der Kopfhaut eine Überempfindlichkeit gegen Testosteron und seinen Metaboliten 5-α-Dihydrotestosteron (5a-DHT) entwickeln. Durch eine Signalübertragung vom 5a-DHT-Rezeptor wird das Enzym Adenylcyclase gehemmt. Adachi (1970) hat herausgefunden, dass die Aktivität von Adenylcyclase bzw. die Konzentration an cyclo-Adenosin-Monophosphat (cAMP) eng mit dem Haarwachstum verknüpft ist. cAMP ist bekannt als second-messenger und aktiviert sowohl die Energiegewinnungskaskaden als auch die Zell-Neogenese durch Aktivierung des Protein-Kinase-Systems. Beide Stoffwechselwege sind für den normalen gesunden Haarwuchs von Bedeutung. Unter einer Mangelsituation leitet der aktive Haarfollikel spontan eine Ruhephase ein, in der das betroffene Haar vorzeitig ausfällt.

Nach einer mehrmonatigen Ruhephasc wächst aus dem Haarfollikel zwar wieder ein neues Haar. Da der Mangelzustand wegen der unverändert fort bestehenden genetisch bedingten Überempfindlichkeit andauert, kann auch die neue IIaarwachstumsphase nicht normal beendet werden. In Folge verkürzen sich die Wachstumszyklen und der Haarfollikel verkümmert und erschöpft schließlich.

Von dieser Entwicklung sind hauptsächlich Männer betroffen, seltener Frauen. Die Bildung der Glatze erfolgt in einem typischen Muster, welches von Hamilton und Norwood in 5 Stadien untergliedert wurde. Bei Frauen tritt der androgenetisch bedingte Haarausfall meist nach der Menopause auf. Er zeigt bei der Frau aber nicht die typischen Verlaufstadien wie beim Mann, sondern tritt in einer diffusen Form auf.

Coffein kann indirekt aufgrund einer fakultativ adrenergen Wirkung Adenylcyclasc aktivieren. Wichtiger ist jedoch, dass Coffein das Enzym Phosphodiesterase hemmen und damit die Spaltung von cAMP verhindern kann. Damit wird der Gehalt an cAMP stabilisiert und so auch die Wachstumsvoraussetzungen für den Haarfollikel. Da das Wirkprinzip von Coffein nicht auf dem Antagonismus eines Androgenrezeptors bzw. dessen Blockade beruht sondern ein indirekt kompetitiver Vorgang von Folgcprozcsscn ist, darf durch den Einsatz von Coffein ein sehr hohes Maß an Verträglichkeit erwartet werden. Die Blockade von Androgen-Rezeptoren ist hingegen nicht regiospezifisch, so dass auch Androgenrezeptoren in anderen Bereichen des Körpers blockiert werden können, was unerwünschte Nebenwirkungen zur Folge haben könnte. Entsprechend wird auch bei Wirkstoffen wie Finasterid, die in der Haarwurzel Androgen-Rezeptoren blockieren sollen, über ein Risiko unerwünschter Wirkungen an Androgen-Rezeptoren anderer Körperregionen berichtet. Finasterid ist bei Frauen im gebärfähigen Alter deshalb streng kontraindiziert.

Die kompetitive Coffeinwirkung an sich ist nicht neu, sondern in verschiedenen Literaturzitate zu finden (Czok, 1976). Daher hat es in der Vergangenheit verschiedene Versuche gegeben, Coffeinlösungen zur Behandlung bzw. Vorbeugung des Haarausfall vom männlichen Typ (Alopecia androgenetica) einzusetzen. So wird in der DE 100 35 735 A1 Coffein in Verbindung mit Fettsäuren und Biotin zur Vorbeugung gegen Haarausfall empfohlen.

In einer weiteren Druckschrift DE 197 01 651 Al wird die Verwendung von Coffein in liposomalen Zubereitungen vorgeschlagen. Üblicherweise werden diese Mittel als topisch applizierbare und auf der Kopfhaut verbleibende Mittel beschrieben. In allen bekannten Schriften wird der besseren Verfügbarkeit wegen das coffeinhaltige Mittel als "Lcavc On" beschrieben. Diese Mittel werden aufgetragen und nicht wieder ausgespült. Durch den pennanenten Kontakt mit der Kopfhaut kann das Coffein in ausreichendem Maße in die Haut bzw. in die Haarwurzel eindringen.

Mitteln, die auf der Kopfhaut verbleiben, können jedoch gewisse Nachteile anhaften. Durch Hilfsstoffe zur Lösungsvermittlung und andere Stabilisatoren kann das Haar durch solche Tonika übermäßig belastet werden. Derart behandeltes Haar macht leicht einen ungepflegten Eindruck, so dass die Anwender-Compliance solcher Produkte nicht besonders ausgeprägt ist.

Gerade die regelmäßige Anwendung ist aber eine wichtige Voraussetzung für die Wirksamkeit. Da die genetisch exponierte Haarwurzel permanent unter dem Einfluss der Androgene steht, muss ebenso dauerhaft diese Wirkung kompetitiv aufgehoben werden. Ein weiterer Nachteil eines Tonikums ist, dass dieses nicht den Haarpflegegewohnheiten des Mannes entspricht. Aus Erfahrung weiß man, dass der Mann möglichst einfach anzuwendende Mittel schätzt.

Daher bestand die Aufgabe darin, ein Mittel gegen erblich bedingten Haarausfall zu schaffen, das in der Anwendung dem Mann bereits gut geläufig ist, so dass die Akzeptanz und damit auch die regelmäßige Anwendung sichergestellt ist.

Diese Aufgabe wird erfindungsgemäß durch die Verwendung eines coffeinhaltigen Mittels, das für den kurzzeitigen Kontakt mit der Kopfhaut bestimmt ist und als Penetrationshilfe anionische und amphotere oberflächenaktive Stoffe (Tenside) sowie die Haarwurzeln stabilisierende Stoffe, insbesondere Minoxidil oder Finasterid, enthält, wobei das Mittel Coffein in einer Menge zwischen 0,01 und 10% enthalt und die Konzentration an anionischen oberflächenaktiven Stoffen (Tenside) zwischen 0,1 und 50% beträgt, vorzugsweise zwischen 1% und 15%, gegen erblich bedingten Haarausfall.

Es liegt im Rahmen der Erfindung, dass das Mittel zusätzlich nichtionische oberflächenaktive Stoffe (Tenside) enthält.

Eine vorteilhafte Ausgestaltung der Erfindung besteht darin, dass die Konzentration an amphoteren oder nichtionischen oberflächenaktiven Stoffen (Tensiden) zwischen 0,5 und 10% beträgt.

Bei einer Weiterbildung der Erfindung ist vorgesehen, dass das Mittel Proteinzusätze, Aminosäuren, Spurenelemente oder Vitamine zur Pflege der Kopfhaut enthält.

Weiterhin kann vorgesehen sein, dass das Mittel Antischuppenwirkstoffe, insbesondere Piroctone Olamine, Zink-Pyrithion, Climbazol, Salicylsäure oder Fumarsäure enthält.

Eine Ausbildung der Erfindung besteht darin, dass das Mittel als Shampoo-Formulierung zusammengesetzt ist.

Bei einer anderen Ausbildung der Erfindung ist vorgesehen, dass das Mittel als Spülungsformulierung mit kationischen Haarpflegestoffen zusammengesetzt ist.

Ebenso ist es zweckmäßig, dass das Mittel als Haar- oder Kopfhautkur aus Lipidstoffen zur Pflege trockener Haare und Kopfhaut zusammengesetzt ist.

Schließlich ist es auch erfindungsgemäß, dass das Mittel entweder als Schaum oder als schäumendes Gel zusammengesetzt ist und als Aerosolanwendung konditioniert ist.

Überraschenderweise wurde im Rahmen der Erfindung gefunden, dass eine Rinse-Off-Formulierung in der Lage ist, in kurzer Zeit den Wirkstoff Coffein in die Haarwurzel zu transportieren. Diese spezifische Rinse-Off-Grundlage wurde als Haarshampoo entwickelt.

Die Haarwäsche ist heute Bestandteil der täglichen Körperhygiene. Gleichwohl besteht beim Mann die Sorge, dass gerade die tägliche Haarwäsche sowohl Kopfhaut und auch Haarwurzel unnötig reizen und damit das Risiko eines vorzeitigen Haarausfalles auch noch steigern könnte. In diesem Zusammenhang war es besonders vorteilhaft, den Wirkstoff Coffein direkt in ein Shampoo einzuarbeiten. Der betroffene Mann genießt es dabei, seine täglichen Haarpflegegewohnheiten beizubehalten und gleichzeitig der Bildung einer Glatze vorzubeugen.

In den Versuchen hat sich gezeigt, dass eine Kombination aus anionischen und amphoteren Tensiden hervorragend geeignet war, die Problemstellung zu lösen. In einem Penetrationsversuch wurde die tensidhaltige Coffeinlösung auf die Haut aufgetragen und nach 2 Minuten Einwirkzeit wieder entfernt. Die nachfolgende Untersuchung der Penetration erfolgte über den Nachweis mit einer spezifischen Sonde im Blut.

In einem zweiten Versuch wurden die Haarfollikel in dem Test-Areal künstlich verschlossen und erneut nach einer Einwirkzeit von 2 Minuten entsprechende Untersuchungen im Blut durchgeführt.

Aus der Differenz der beiden Versuchsreihen konnten kinetische Betrachtungen zur Penetrationsroute angestellt werden.

Überraschenderweise zeigte sich, dass schon nach 5 min über den follikulären Penetrationsweg Coffein im Blut nachweisbar war. Das Maximum war bereits nach 20 min erreicht. Die dermale Route zeigte erst mit deutlicher Verzögerung nennenswerte Coffein-Werte im Blut an.

Dieses Ergebnis übemaschte, da bisher nur bekannt war, dass Wirkstoffe in tensidhaltigen Rinse-Off-Zubereitungen entweder nur auf das Haar aufziehen konnten (sog. FIaarkonditionierer) oder sich auf der Oberfläche der Kopfhaut abgeschieden haben (z.B. Antischuppenmittel). Eine rasche Penetration und damit biologische Verfügbarkeit in der Haarwurzel war wegen der kurzen Kontaktzeit ausgeschlossen worden.

In der Grafik 1 ist die gesamte dermale Resorptionskinctik einschließlich der follikulären Route dargestellt. In Grafik 2 ist die rein follikuläre Resorptionskinetik herausgestellt.

Danach zeigt sich, dass eine anionisch-amphotere Tensidmischung eine spezifisch penetrationsfördernde Eigenschaften für Coffein besitzt. Der genaue Mechanismus bedarf dabei noch weiterer Untersuchungen. Die rasche Resorption wird auf eine ionische Wechselwirkung zwischen dem basischen Coffein und dem anionischen Tensid zurückgeführt.

Der erfindungsgemäße Gegenstand kann in einer Shampoo-Formulierung eingesetzt werden und dabei noch durch zusätzliche Wirkstoffe ergänzt werden.

Als anionische Tenside kommen folgende Stoffgruppen in Frage: Alkylethersulfate, Alkylsulfate, Alkyl-Sulfosuccinate, Ethercarbonsäuren oder Estercarbonsäuren, Alkylsarkosinate, -glutamate, - glycinate und -taurate, Alkylphosphate oder Alkylsulfonate.

Ergänzt werden die anionischen Tenside durch amphotere Tenside, Alkylamidobetaine und Alkylbetaine, wie z.B. Cocamidopropylbetaine, Amphoacetate oder Amphodiacetate, wie z.B. Cocoamphodiactetatc, Cocamide MEA oder -DEA und nichtionische Tenside. Beispielhaft seien Alkylglycoside und Alkoxyglycoside genannt.

Insbesondere sind Kombinationen mit sekundären Pflanzeninhaltsstoffen aus Chamomilla, Hypericum, Ginkgo, Tussilago, Berberis, Camellia, Glycyrrhiza, Humulus möglich, die reizlindernd und antiirritativ wirksam sind.

Ebenso sind Zusätze aus hydrolysierten Proteinfraktionen pflanzlicher oder tierischer Quellen möglich. Die Zusätze können zum Beispiel aus Weizen, Hafer, Seide, Kollagen, Keratin, Milchprotein, Soja, biotechnologisch gewonnenem Pseudokollagen etc. stammen. Sie werden eingesetzt, um Strakturschäden am Haar zu beseitigen oder als sogenannte Feuchthaltestoffe. Ergänzend können Stoffe wie Panthenol, Allantoin, Glycerin, Glycole, Sorbit eingesetzt werden.

Vorteilhaft für die Pflege der Kopfhaut sind Zusätze von Vitaminen, z.B. Niacin, Biotin, Vitamin E, Vitamin C oder Coenzym Q10.

Weiterhin bewährt sich der Zusatz von Spurenelementen, wie z.B. Zink, Eisen, Mangan, Kupfer.

Zur gleichzeitigen Beseitigung von Kopfschuppen können z.B. Piroctone Olamine, Salicylsäure, Fumarsäure, Climbazol oder Zink-Pyrithion eingesetzt werden.

Zur Haarpflege insbesondere poröser Haare können kationische Pflegestoffe in monomerer als auch polymerer Form vorliegen. Beispielhaft sei hier genannt Cetrimonium Chlorid; als polymere Pflegestoffe gelten Polyquaternium-10.

Lichtschutzfilter, um das Haar vor LTV-Strahlung zu schützen, sind ebenfalls von Nutzen. Als Vertreter dieser Wirkstoffgruppe gelten beispielsweise Benzophenone, p-Aminobenzoesäure, Zimtsäurederivate, Bis t-Butyl Dibenzoylmethan, 4-Methyl Benzylidencampher.

Auch pflanzliche oder synthetische Öle zur Verbesserung des Glanzes und der Geschmeidigkeit des Haares sind nützlich. Typische pflanzliche Öle sind Sojaöl, Sonnenblumenöl, Mandelöl, Traubenkernöl, Avocadoöl, Jojobaöl, aus synthetischer Herkunft modifizierte Silikonöle des Typs Dimethicone und Dimethiconol, auch als kationische Derivate wie Amodimethicone.

Zur Stabilisierung werden Komplexbildner wie NTA, EDTA, Phytinsäure, Polyphosphate, HEDP, Citronensäure, Weinsäure und andere Polyhydroxycarbonsäuren eingesetzt.

Weitere Stabilisatoren sind Antioxidationsmittel wie BHT, BHA und Vitamin E Acetat.

Zur Vermeidung mikrobiollen Verderbs bei der Anwendung werden organische Säuren wie Ameisensäure, Sorbinsäure oder Benzoesäure vorgeschlagen oder Ester der p-Hydroxybenzoesäure, Formaldehyd abspaltende Konservierungsstoffe wie DMDM Hydantoin, Imidazolidinylharnstoff, oder Methyl Chloroisothiazolinon, Methylisothiazolinone, Dibromodicyanobutane, Iodopropynyl Butylcarbamate, Phenoxyethanol oder Benzylakohol.

Zur Stabilisierung des pH-Wertes werden Puffersalze mehrwertiger organischer Säuren und deren Salze wie Citronensäure, Weinsäure oder Äpfelsäure eingesetzt.

Zur Verbesserung der Konsistenz haben sich nichtionische Verdicker, sogenannte Alkylethoxylatc bewährt.

Abschließend ohne Nennung detaillierter Beispiele sei ausgeführt, dass zur Solubilisierung wasserunlöslicher Wirkstoffe auch Alkohole, ein- oder mehrwertig eingesetzt werden können, ebenso Methyl- oder Ethylether mehrwertiger Alkohole oder Polyglycerine oder Polyglycole.

Als geeignete Rinse-Off Rezeptur ist auch eine Coffein-Spülung oder Coffein-Haar-Kur möglich. Im Gegensatz durch das Shampoo ist bei einer Haar-Kur durch die etwas längere Einwirkzeit sogar noch eine verstärkte Wirkung zu erwarten. Der Einsatz in einer Kur ist auch insofern vorteilhaft, weil der Kunde eine erkennbaren Zusatznutzen erzielt. Da Kuren bisher in der Regel zur Verbesserung der Struktur des äußerlichen Haarschaftes eingesetzt werden, könnte durch eine Coffein-Kur die Haarbeschaffenheit von Grund auf durch die Stabilisierung der Haarwurzel erzielt werden.

Zur Verdeutlichung der Vielfalt an Rezepturvarianten sollen nachfolgende Rahmenrezepturen dienen:

### Formel A: Haarshampoo mit kombinierter Kopfhautwirkung gegen Schuppen

| | Gew% |
|---|---|
| Sodium Laureth Sulfate | 10% |
| Cocoamphoacetate | 2% |
| Glycerin | 2% |
| Panthenol | 0,5% |
| Hydrolyzed Collagen | 1,0% |
| Sodium Polyphosphate | 0,1% |
| Parfum | 0,3% |
| Sodium Chloride | 0,3% |
| Piroctone Olaminc | 0,5% |
| Caffieine | 0,3% |
| Formic Acid | 0,2% |
| Aqua | ad 100 |

### Formel B: Haarshampoo für feines Haar

| | |
|---|---|
| Sodium Lauryl Sulfate | 4% |
| Sodium Laureth Sulfate | 5% |
| Hydroxypropyltrimonium Hydrolyzed Wheat Protein | 1% |
| Disodium Laureth Sulfosuccinate | 3% |
| Biotin | 0,02% |
| Tocopherylacetate | 0,3% |
| Citric Acid | 0,2% |
| Caffeine | 0,5% |
| Parfum | 0,3% |
| Potassium Sorbate | 0,2% |
| DMDM Hydantoin | 0,1% |
| Aqua | ad 100 |

### Formel C: Haarspülung

| | Gew % |
|---|---|
| Sodium Laureth-11 carboxylate | 1,0 |
| Cetrimonium Chloride | 2% |
| Steareth-10 | 5% |
| Cetearyl Acohol | 4% |
| Parfum | 0,5% |
| Caffeine | 0,4% |
| Hydrolyzed Keratin | 0,5% |
| Citric Acid | 0,2% |
| Sodium Benzoatc | 0,05% |
| Potassium Sorbate | 0,15% |
| Aqua | ad 100 |

### Formel D: Haar- und Kopfhaut-Kur

| | |
|---|---|
| PEG-9 Stearamide Carboxylic Acid | 2% |
| Cetyl Alcohol | 6% |
| Panthenol | 1% |
| Caffeine | 0,5% |
| Polyquaternium-10 | 0,5% |
| Dimethiconol | 2% |
| Phytic Acid | 0,5% |
| Bisabolol | 0,2% |
| PEG-25 PABA | 0,5% |
| Aqua | ad 100 |

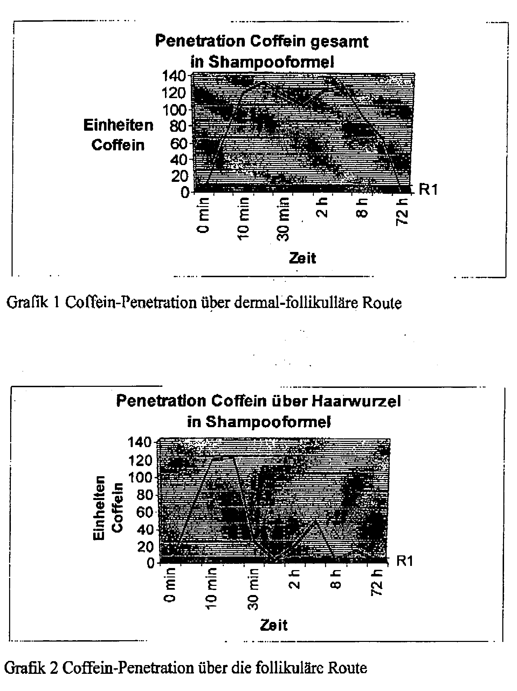

## Patentansprüche

1. Verwendung eines eoffeinhaltigen Mittels, das für den kurzzeitigen Kontakt mit der Kopfhaut bestimmt ist und als Penetrationshilfe anionische und amphotere oberflächenaktive Stoffe (Tenside) sowie die Haarwürzeln stabilisierende Stoffe, insbesondere Minoxidil oder Finasterid, enthält, wobei das Mittel Coffein in einer Menge zwischen 0,01 und 10% enthält und die Konzentration an anionischen oberflächenaktiven Stoffen (Tenside) zwischen 0,1 und 50% beträgt, vorzugsweise zwischen 1% und 15%, gegen erblich bedingten Haarausfall.

2. Verwendung gemäß Anspruch 1, **dadurch gekennzeichnet, dass** das Mittel zusätzlich nichtionische oberflächenaktive Stoffe (Tenside) enthält.

3. Verwendung gemäß Anspruch 2, **dadurch gekennzeichnet, dass** die Konzentration an amphoteren oder nichtionischen oberflächenaktiven Stoffen (Tensiden) zwischen 0,5 und 10% beträgt.

4. Verwendung gemäß Anspruch 1, **dadurch gekennzeichnet, dass** das Mittel Proteinzusätze, Aminosäuren, Spurenelemente oder Vitamine zur Pflege der Kopfhaut enthält.

5. Verwendung gemäß Anspruch 1, **dadurch gekennzeichnet, dass** das Mittel Antischuppenwirkstoffe, insbesondere Piroctone Olamine, Zink-Pyrithion, Climbazol, Salicylsäure oder Fumarsäure, enthält.

6. Verwendung gemäß Anspruch 1, **dadurch gekennzeichnet, dass** das Mittel als Shampoo-Formulierung zusammengesetzt ist.

7. Verwendung gemäß Anspruch 1, **dadurch gekennzeichnet, dass** das Mittel als Spülungsformulierung mit kationischen Haarpflegestoffen zusammengesetzt ist.

8. Verwendung gemäß Anspruch 1, **dadurch gekennzeichnet, dass** das Mittel als Haar-oder Kopfhautkur aus Lipidstoffen zur Pflege trockener Haare und Kopfhaut zusammengesetzt ist.

9. Verwendung gemäß den Ansprüchen 5 bis 7, **dadurch gekennzeichnet, dass** das Mittel entweder als Schaum oder als schäumendes Gel zusammengesetzt ist und als Aerosolanwendung konditioniert ist.
